# EUROPEAN PATENT APPLICATION

(11) **EP 4 763 168 A1**
(43) Date of publication of application: **24.06.2026**
(21) Application number: 24221218.1
(22) Date of filing: 18.12.2024
(51) Int. Cl.: A61F 13/49, A61F 13/505

(54) **WASHABLE ABSORBENT ASSEMBLY**

(71) Applicant: Essity Hygiene and Health Aktiebolag, 405 03 Göteborg (SE)
(72) Inventor: KNÖS, Anna, 405 03 GÖTEBORG (SE); PALMQVIST, Lisa, 405 03 GÖTEBORG (SE); BLOMSTRÖM, Philip, 405 03 GÖTEBORG (SE)
(74) Representative: Essity Hygiene and Health AB

(57) **Abstract**

A washable integrated absorbent assembly for an absorbent article comprising an absorbent layer and a liquid barrier. The absorbent layer is of a knitted material having first and second surfaces, the first surface having a loop structure. The knitted absorbent material comprises a major amount of viscose, and further comprises cotton and polyester.

## Description

### Field

The present disclosure relates to a washable integrated absorbent assembly for an absorbent article comprising a knitted absorbent material and a liquid barrier.

### Background

A washable absorbent article, such as a washable absorbent underwear or a washable absorbent pad, may be worn for the purpose of absorbing body fluids such as urine and vaginal fluids. Such garments form an environmental and economical advantageous alternative to disposable sanitary napkins and disposable absorbent undergarments. A washable absorbent article typically comprises fabrics of woven or knitted materials. Conventionally such a washable absorbent article will also comprise an absorbent assembly for absorbing body fluids, sometimes referred to as the gusset, located in the crotch region of the wearer when the article is worn. After use the article is washed or laundered before reuse. An example of a washable absorbent undergarment is found in WO2023/057096A1.

Depending upon the intended use, the absorbent assembly may vary in size and construction. It may extend over a smaller or larger area of the garment, e.g. depending on whether it is intended for daytime or night-time use. It may also be thicker or thinner, with additional wicking, distribution, barrier, and absorbing layers. Various materials have been used for these washable absorbent articles. Natural and untreated materials are desired for such articles for sustainable reasons but may have different absorption properties than treated or synthetic materials and may thus not fulfill looked-for absorption criteria.

The selection of absorbent materials for incorporation into the absorbent assembly is one of the major challenges in developing washable and reusable absorbent articles. It would thus be desirable to find materials that improve the absorbent efficacity of a washable absorbent assembly.

### Summary

This object may be at least partially achieved with a washable absorbent assembly in accordance with claim 1 and a washable absorbent undergarment according to claim 14. Further embodiments are set out in the dependent claims and in the following description.

The disclosure thus concerns a washable integrated absorbent assembly for an absorbent article, the assembly extending in a longitudinal direction and having a transverse direction and comprising an absorbent layer and a liquid barrier. The absorbent layer being of a knitted material having a first and a second surface, the first surface having a loop structure. The knitted absorbent material comprises a major amount of viscose and further comprises cotton and polyester.

It has been found that the inventive knitted absorbent material has an unexpected combination of both a high absorption and a high holding capacity compared with known materials and material combinations used in the area.

The knitted absorbent material comprises a major amount of viscose and further comprises cotton and polyester. A major amount of viscose means that more than 50% by weight of the material in the absorbent layer is of viscose. The knitted absorbent material may comprise 60-85% by weight viscose. The absorbent material may specifically comprise at least 60-85% by weight viscose, 10-25 % by weight cotton and 5-20 % by weight polyester, such as 65-80% viscose, 10-20% Cotton, 5-15% Polyester.

The knitted absorbent material may be a weft knit or a circular knit. The absorbent layer is a knitted material having a first surface having a loop structure and a second surface opposite said first surface may have rows of V-shaped stitches. The first loop surface may comprise viscose yarn and the second surface may comprise cotton and polyester yarn. The loops may have a mainly horizontal orientation on the material. The knitted absorbent material may be a one-sided or double-sided stretch Terry. The layer of absorbent material may have a basis weight of 180-600 g/m². This basis weight may be achieved by a single layer of absorbent material although it is not excluded that multiple layers may be provided to increase the basis weight.

The absorbent material of viscose, cotton and polyester may be without functional treatments such as wetting agents, softening agents, anti-odor treatments, optical brighteners, and biocidal agents.

The absorbent assembly according to this disclosure is incorporated into a washable absorbent underwear. The assembly may be permanently attached to the body panel of the underwear.

The yarns forming the absorbent layer may have any appropriate construction or weight to achieve the desired absorption capacity. The yarns may include spun yarns of short staple fibres and may be single-ply or multi-ply. The yarns may be relatively fine to favour a close knit that will not easily unravel. The yarn count may be 45-65 S. One yarn may be of viscose and the second yarn cotton/polyester 60:40 in yarn.

The absorbent assembly may comprise further layers, such as a wearer facing top layer, wicking layer(s) and one or more further absorbent layers.

The further layers may comprise a wearer facing top layer. The top layer may comprise a water-permeable material allowing the body fluids to migrate to the underlying absorbent layer. The top layer may be constructed of any suitable fabric, including natural and/or naturally derived fibres selected from the group consisting of cotton, wool, silk, cellulose, regenerated cellulose, rayon, viscose, modal, lyocell, Tencel, bamboo, hemp, flax, ramie, coir, or banana. Alternatively, the top layer may be constructed of synthetic fibres selected from the group consisting of polyamide, acrylic, polyester, or elastane, such as a mixture of polyester and elastane. Further, the top layer may be constructed of a blend or a mixture of naturally derived and/or synthetic fibres. The materials used for construction of the top layer should be soft and non-irritating to the skin and be readily penetrated by any body fluids. The top layer is washable and may be of a textile material, such as a knitted material. The top layer may comprise between 20% and 100% natural or naturally derived fibres, such as between 40% and 100% natural or naturally derived fibres and such as between 60% and 100% natural or naturally derived fibres. The top layer may have a basis weight of 80-200 gsm.

Further layers may comprise a moisture distributing orwicking layer, which may be provided directly underneath the top layer. The wicking layer may have wicking features to allow the moisture to spread away from the wearer and into the absorbent layer. Wicking enables an efficient spread of the moisture, allowing the moisture to be received into a wider area of the underlying absorbent layer. This feature is particularly relevant for users suffering from stress incontinence as spurts of urine may cause the absorbent layer to be saturated rapidly at the point of impact. This function is also very important when the absorbent layer comprises isolated or partially isolated segments or regions, whereby wicking and fluid transmission between segments/regions is reduced. By spreading the volume of the fluid over a wider receiving area in the absorbent layer, the wicking features of the wicking layer increase the overall absorptive capacity of the absorbent layer. The wicking layer may be constructed of any suitable fabric, including natural and/or naturally derived fibres or mixtures thereof selected from the group consisting of cotton, wool, silk, cellulose, regenerated cellulose, rayon, viscose, modal, lyocell, Tencel, bamboo, hemp, flax, ramie, coir or banana. Alternatively, the wicking layer may be constructed of synthetic fibres or mixtures thereof selected from the group consisting of polyamide, acrylic, polyester, or elastane. Further, the wicking layer may be constructed of a blend or a mixture of natural, naturally derived and/or synthetic fibres. The wicking layer should be washable and of a textile material, such as a knitted material. The wicking layer may comprise between 20% and 100% natural or naturally derived fibres, such as between 40% and 100% natural or naturally derived fibres, and such as between 60% and 100% natural naturally derived fibres. The basis weight of the wicking layer may be 180-250 gsm.

The moisture barrier layer functions to prevent moisture from penetrating from the absorbent layer to the garment fabric. The moisture barrier layer may be an extruded layer. Suitable moisture impermeable materials may include thermoplastic films, such as polyurethane or the like. Alternatively, it may comprise a weave or knit. Certain filaments and yarns are naturally hydrophobic and a closely woven or knit structure can thus achieve significant water repellence without need for additional coatings. The moisture barrier layer may also comprise a moisture impermeable material laminated or coated onto a woven or knitted material. The moisture barrier layer may have a surface weight of between 50 and 300 gsm, such as between 75 gsm and 200 gsm. Although the moisture barrier layer may be impervious to liquid water, the moisture barrier layer may be breathable. It may have a breathability or moisture vapor transmission rate (WVTR) of more than 500g. WVTR is measured by the rate at which water vapor passes through, in grams of water vapour per square meter of fabric per 24-hour period (g/m2/d), often abbreviated to just "g". The barrier layer should be launderable.

The absorbent layer may be connected to the one or more further layers in any suitable manner. The absorbent layer may be connected to the one or more further layers at its side edges, which may also correspond to the edges of the assembly. This may be sufficient in cases where the absorbent layer extends the full width of the assembly without interruption. The absorbent layer may be connected to the one or more further layers only at its longitudinal side edges. It may also or alternatively be connected to the one or more further layers at its transversal ends. The layers may be connected by stitches, staples, adhesive, welding, tape or the like. The absorbent layer may be connected to just one of the further layers, e.g. to the top layer or wicking layer that is at the wearer facing side. Alternatively, it may be connected to further layers on either side of it. The absorbent layer may be connected to all the layers of the assembly and/or to fabric panels of the undergarment.

The assembly may have an area adapted to the size of the undergarment and the intended use. The assembly may have an area of at least 10 cm², such as between 10 cm² and 400 cm², or between 50 cm² and 100 cm². The absorbent layer may have similar area and may be co-extensive or substantially co-extensive with the assembly over the whole area of the assembly. The absorbent layer may occupy at least 40 % of the area of the assembly or more than 90 % or more than 95% of the area.

The disclosure also relates to a washable absorbent undergarment comprising one or more fabric panels and an assembly as described above and hereinafter located in a crotch region.

As used herein, the term "absorbent undergarment" refers to garments that are worn and intended to be placed against the skin of the wearer with the intended function of absorbing and containing body fluids such as urine and vaginal fluids including menstrual fluid, without significant visible external trace. The undergarment may for example be underwear, underpants, a panty, or swimwear of fabric. The undergarment is intended for teenage and adult users. The undergarment may be designed for male or female use. It will be understood that most conventional garments will have a limited degree of absorbency but that for conventional undergarments the containment of body fluids is not intended, and an unexpected leakage of any significant volume would be perceptible externally either as a damp patch or discoloration that may penetrate through an outer garment. The material of the undergarment may be of natural or synthetic materials, or a mixture thereof.

The absorbent undergarment and the absorbent assembly according to the present disclosure is washable, i.e. intended to be laundered or otherwise restored after use for repeated reuse as a sanitary article. By "washable" it is meant that the absorbent undergarment may be cleaned by laundering. The absorbent undergarment may thus be subjected to an aqueous solution containing detergent without losing its structural features. This aqueous solution may be heated as part of the laundering process, e.g. to 30°C or 60°C. The term is also intended to mean that the absorbent undergarment can be washed and reused more than once. Washable may be understood as being suitable to undergo at least 10 washing cycles, such as at least 50 washed at a temperature of at least 30°C, without disintegrating. During the washing, the absorbent assembly releases the absorbed fluids, thereby regenerating the absorptive functioning, enabling the absorbent assembly to be reused for the same purpose again. A disposable absorbent article is not constructed to be washable and reusable and is thus for single use only.

The term "fabric" as used in the present disclosure may refer to single or multiple layers of a flexible planar substance constructed from solutions, fibers, yarns, fabrics, or any combination. Fabrics include knitted or woven materials. The term textile refers to any flexible material that is composed of thin films or of fibers, yarns, or fabrics or products made of films, fibers, yarns, or fabrics. Although other terms like material, fabric, and cloth are synonyms, textile is most used in the global textile complex. Fiber is any substance, natural or manufactured, with a high length-to-width ratio with suitable characteristics for processing into fabrics and is the smallest component, hairlike in nature, that can be removed from a fabric. Fibers make most fabrics. A yarn is a grouping of fibers that is twisted or laid together to form a continuous strand that can be made into a textile fabric.

The assembly may be permanently attached to at least one of the one or more fabric panels of the undergarment. By "permanently attached", is meant that no portion is intended to be separated from the fabric panel during, or after use. All portions of the assembly are thus intended to be laundered together with the garment. The assembly may alternatively be configured as a pad that is adapted for detachable connection to the panels of an undergarment. Thereto the assembly may comprise an adhesive layer or a hook patch on its garment facing surface or any other suitable manner of connection. Alternatively, the garment may be provided with a suitable pocket or recess for receiving the assembly.

The assembly may be attached to the fabric panels of the undergarment over its whole surface e.g. by lamination or adhesive. In other embodiments, the absorbent assembly may not be directly bonded to the fabric panels but instead may be attached to the fabric via auxiliary parts such as edging strips or reinforcement patches. In certain embodiments, the absorbent assembly is attached to the one or more fabric panels by one or more bonding members. These may include one or more of a front bond member, a rear bond member and side bond members.

The undergarment may be of any suitable form and reference above and in the following to 'garment' is not intended to mean other than an undergarment i.e. an innermost garment intended for being in contact with the body of a user. The garment has a waist opening and two leg openings. It is however not excluded that closure elements e.g. between the waist opening and the leg openings may be provided, allowing easy removal, or donning of the garment. The undergarment may have various designs, such as briefs, boxer, hipster, high waist, string, Brazilian, or other suitable and conventional undergarment designs. The fabric panels may form a front region, a back region and an intermediate region extending between the front and back regions, the front region and the back region being joined at the sides such that the undergarment forms a waist opening and a pair of leg openings. A central longitudinal axis of the undergarment is defined along the one or more fabric panels of the undergarment from the back region and towards the front region and a transversal crotch axis of the undergarment is defined in a direction extending between the leg openings. The transversal crotch axis may be located at the narrowest point of the garment and/or may be located at the mid-point of the central longitudinal axis. The transversal crotch axis divides the intermediate region into a front intermediate region extending longitudinally between the transversal crotch axis and a front end of each leg opening, and a rear intermediate region extending longitudinally between the transversal crotch axis and a rear end of each leg opening. The transversal crotch axis is perpendicular to the central longitudinal axis and the absorbent layer and further layers are superimposed along a height axis, that is perpendicular to the longitudinal axis and the transversal crotch axis.

The assembly may be in the intermediate region. It may also be located only in the intermediate region i.e. it may not extend into the front region and back regions, which together form the waist region. The assembly may be substantially symmetrical along the central longitudinal axis.

The shape and size of the assembly may be such that at least a crotch section of said undergarment is covered during use. The crotch section is a surface section having a size and shape to cover part of, or the whole, genital area of a user, i.e. a surface section shaped to fit in between the legs of a user, that has a length sufficient to extend at least from the mons pubis to the perineum and a width that varies along the length. The crotch section thus has a width substantially corresponding to a transversal width in between leg openings of an undergarment. Thus, the entire crotch section is protected from soiling by the assembly.

The area of the garment in which the assembly is present may be adapted according to the garment and the intended use. The assembly may cover an area of at least 10 cm² of the garment, preferably between 10 cm² and 400 cm², or between 50 cm² and 100 cm².

The assembly may be of any appropriate shape according to the intended use and the shape of the undergarment. It may be dog-bone shaped, having curved sides, and slightly enlarged ends. It may be four-sided, having a front edge, a rear edge and two side edges. The side edges may be positioned to be coincident with respective leg openings. In this context, "coincident" is intended to mean substantially aligned with and adjacent to the side edges, within the bounds of normal manufacturing practices. The assembly or parts thereof may wrap around the side edges in a seam or vice-versa. The assembly may also end just short of the side edges and be covered by an edging strip. The absorbent layer may have the same size and shape as the assembly or may have the same shape but be marginally smaller in size.

The assembly may be rectangular. In this context, "rectangular" is intended to denote that the assembly has an outer contour that is a four-sided shape, having two pairs of sides that are generally parallel to one another. The rectangular assembly or its components can be easily cut multiple times from a main material source (often a large sheet or roll of material), with a single cutting line forming two edges of adjacent components. Thus, the use of rectangular shaped assemblies reduces the amount of cutting and minimizes waste. Furthermore, the cutting of rectangular contours from a main material source allows for multiple components to be cut directly adjacent to one another, without having to account for a positioning direction of each individual component in the manufacturing line. Thus, no energy is wasted during production to account for variation in direction of each component.

At least 75% of the total length of the edges of the at least one rectangular shaped assembly may be straight edges. Thus at least 75% of the outer contour of the rectangular shaped assembly conforms to the rectangular shape, thereby limiting the amount of material wasted, while allowing for some minimal shape variations in the layer that are required to assure wearer comfort and prevent the article from bulging during use.

The one or more further layers may all be substantially equal in size and shape and may all have an outer contour that forms an outer contour of the assembly. In this manner, protection against leakage is available over the same area that is also directly visible to the user, giving a good indication to the user of the leakage protection provided by the assembly. In other embodiments, the layers need not be co-extensive. A top layer may extend beyond the moisture barrier layer e.g. to connect to the fabric panels of the garment.

### Brief description of the drawings

Embodiments of a washable absorbent undergarment according to the present disclosure will be described by way of example, with reference to the attached drawings, in which:
Fig. 1 shows a front view of an exemplary absorbent undergarment comprising an absorbent assembly;
Fig. 2 shows a top view of the undergarment of Fig. 1 in a flat laid-out state with the joints between the rear region and the front region removed;
Fig. 3 is a schematic cut-away view of a portion of the absorbent undergarment of Fig. 2;
Fig. 4 is a cut-away perspective view through the undergarment of Fig. 1 in the crotch region;
Fig. 5 is a detail of the absorbent layer of Fig. 4;

Different aspects of the present disclosure will be described more fully hereinafter with reference to the enclosed drawings. The absorbent undergarment disclosed herein can, however be realized in many different forms, such as different sizes and absorption levels, and should not be construed as being limited to the aspects set forth herein. In all figures of the following detailed description, the same reference numerals will be used to indicate the same elements.

### Description

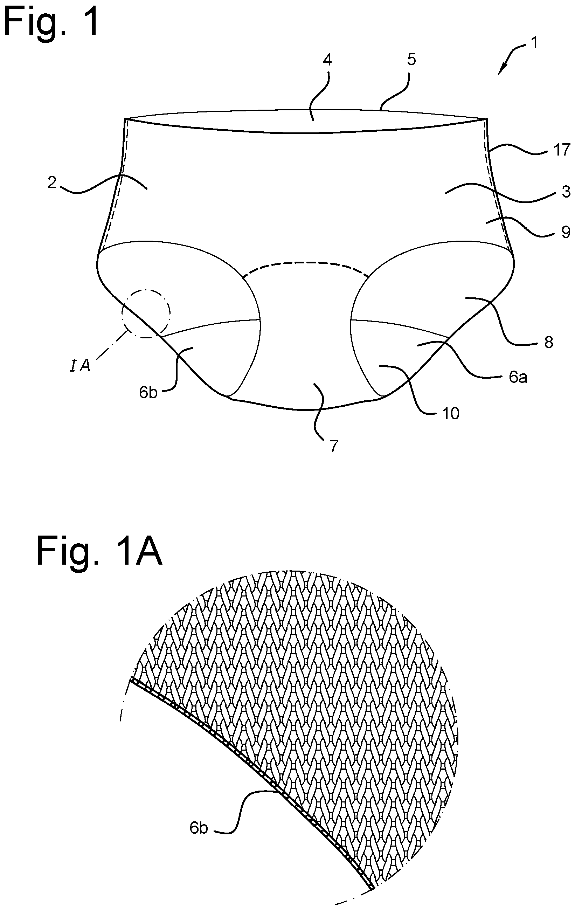

Fig. 1 illustrates as an example a washable and reusable absorbent undergarment 1. As outlined in the above, the undergarment 1 as proposed herein may however have various designs, such as briefs, boxer, hipster, high waist, string, Brazilian, or other suitable undergarment designs. The undergarment 1 may be designed for male or female use.

The undergarment 1 comprises one or more fabric panels 2 forming a front region 3, a back region 4 and an intermediate region 7 extending between the front and back regions 3, 4. The front region 3 and the back region 4 are joined such that the undergarment 1 forms a waist opening 5 and a pair of leg openings 6a, 6b. As such, the front region 3 will be visible from the front of the user when the undergarment 1 is worn, and the back region 4 will be visible from the back of the user when the undergarment 1 is worn. The undergarment of the Fig. 1 example comprises one fabric panel 2, which is cut to form the front region 3, the back region 4 and the intermediate region 7. However, in other variants, a plurality of fabric panels may be joined to form the front region 3, the back region 4 and the intermediate region 7. For example, the undergarment 1 may comprise a front panel, a back panel, and an intermediate panel. The one or more fabric panels may all comprise the same fabric, giving the undergarment a unitary appearance, or the one or more panels may comprise different fabrics, for example to provide aesthetically pleasing undergarments comprising e.g. lace fabric.

The fabric of the one or more fabric panels 2 may be any suitable fabric, including naturally derived fibres and mixtures thereof selected from the group consisting of cotton, wool, silk, cellulose, regenerated cellulose, rayon, viscose, modal, lyocell, Tencel, bamboo, hemp, flax, ramie, coir, or banana. Alternatively, the fabric may be constructed of synthetic fibres or mixtures thereof selected from the group consisting of polyamide, acrylic, polyester. Further, the fabric may be constructed of a blend or a mixture of naturally derived and/or synthetic fibres. The fibres may be recycled fibres. The fabric may comprise a stretchable fabric, e.g. elastane, so that the absorbent undergarment can provide a firm fit while at the same time adapting to the wearer's movements thus preventing any leakage from migrating through the leg openings and keeping the absorbent assembly in place. The fabric may be breathable to allow vapor to escape from the wearer's skin.

In the illustrated embodiment, the material of the fabric panel is a single layer of interlock knitted fabric, Fig. 1 and Fig. 1A, that terminates at the waist opening 5, without any waist band or additional elastic. There is also no seam, elastic or stitching around the leg openings 6a, 6b. A characteristic of interlock knitted fabric is that it can be cut, and the cut edge is stable against fraying without requiring any form of seam. It will nevertheless be understood that in other embodiments, the absorbent undergarment 1 may comprise a waist band, arranged along the waist opening 5 of the absorbent undergarment 1, which may be elasticated and/or provided with a frictional inner surface.

To join the front region 3 and the back region 4 to form the waist opening 5 and the leg openings 6a, 6b, a pair of side joints 17 may be provided, as in the illustrated example. The side joints 17 and any other joints joining the one or more fabric panels 2 may be conventional joints in the art, such as seams made by conventional adhesive and/or conventional mechanical bonds, such as stitching techniques. As illustrated in Fig. 1, the fabric panel 2 has an outside surface 9 facing away from the wearer and an inside surface 8 facing in a direction towards the wearer. An absorbent assembly 10 is located in the crotch region 7 at the inside surface 8.

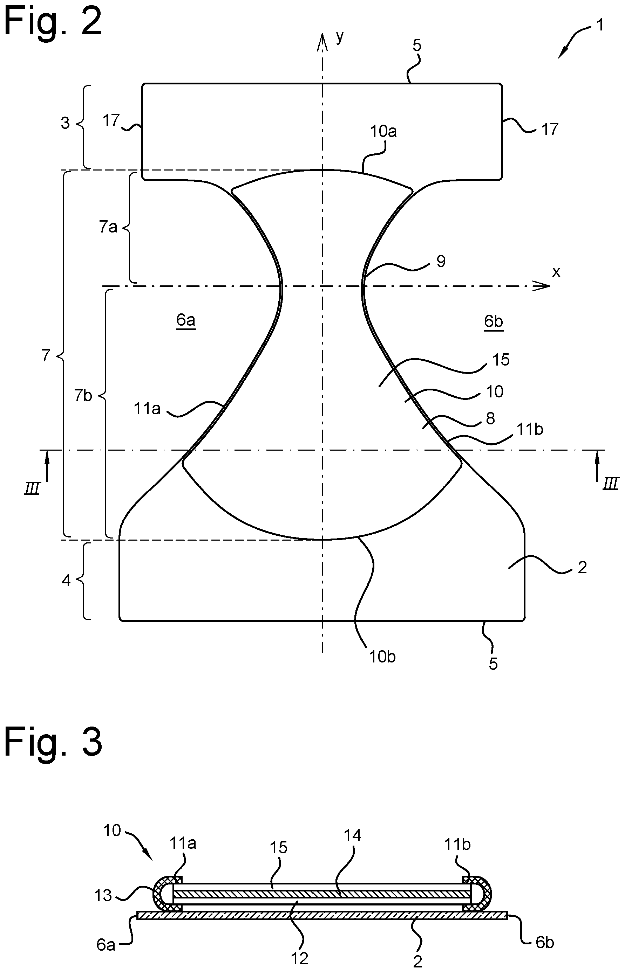

In Fig. 2, the absorbent undergarment 1 of Fig. 1 is shown in a flat laid-out state, where the side joints 17 of the undergarment 1 are removed. As seen in this laid-out state, a central longitudinal axis y is defined along the one or more fabric panels 2 and in a direction from the rear side 4 of the undergarment 1 towards the front side 3 of the undergarment 1. In Fig. 2, the absorbent undergarment 1 is shown from a wearer- facing side of the article 1. As illustrated in Fig. 1, the one or more fabric panels 2 will have an exterior side 9 facing away from the wearer, and an interior side 8 facing in a direction towards the wearer. Fig. 2 thus displays the undergarment 1 in a flat laid-out state as seen from the interior side 8. Further, a transversal crotch axis x of the undergarment 1 is defined in a direction extending between the leg openings 6a, 6b, the transversal crotch axis x being perpendicular to the central longitudinal axis y at the crotch, being the position of minimum distance between the leg openings 6a, 6b.

The assembly 10 comprises a wearer facing top layer 15 that visibly illustrates to a user the extent of the assembly 10. The transversal crotch axis x divides the crotch region 7 into a front assembly region 7a extending longitudinally forwards from the transversal crotch axis x to a front edge 10a of the assembly 10, and a rear assembly region 7b extending longitudinally between the transversal crotch axis x and a rear edge 10b of the assembly 10. The longitudinal extension of the assembly 10 may depend for example on the design of the undergarment 1.

The assembly 10 thus defines the crotch region 7, being generally at a location where the need for absorbance is most prevalent. For undergarments intended primarily for night-time use, the assembly 10 may extend further rearwards and more of the assembly 10 may be located to the rear of the transversal crotch axis x extending further upwards towards the waist opening 5.

As exemplified by the illustrated undergarment 1, the assembly 10 also forms a pair of side edges 11a, 11b. Each side edge 11a, 11b is at least partly directed towards a respective leg opening 6a, 6b. Each side edge 11a, 11b, thus connects the front edge 10a and the rear edge 10b. Each side edge 11a, 11b is arranged to follow the contour of the respective leg opening 6a, 6b of the undergarment 1.

The skilled person will recognise that the shape of the assembly 10 may alternatively be adapted to various absorption needs and to various designs of the undergarment 1, to provide sufficient absorption and satisfactory fit. For example, the side edges 11a, 11b may be straight or may comprise concave portions, as seen towards the central longitudinal axis y. Thus, the assembly 10 may be better adapted to fit between the wearer's legs. The front edge 10a of the assembly 10 may be curved convex as shown. This may be beneficial for the fit of the undergarment to the body and contribute to the avoidance of unwanted creases in the fabric panels. The front edge 10a may also be straight to reduce production and material costs. The same may apply to the rear edge 10b.

Fig. 3 shows a cross-section through the rear assembly region 7b, taken in the direction III-III in Figure 2. The fabric panel 2 can be seen to comprise a single layer of interlock knitted fabric without any seam or other reinforcement at the leg openings 6a, 6b. Beneath the top layer 15 is the absorbent layer 14 and a moisture barrier layer 12. The absorbent layer 14 is co-extensive with the barrier layer 12, and the top layer 15. The top layer 15, the absorbent layer 14 and the barrier layer 12 are connected around the side edges 11a, 11b and the front and rear edges 10a, 10b by single sided adhesive tape 13. The assembly 10 is attached to the fabric panel 2 by adhesive. It will be understood that other methods of joining the layers of the assembly 10 may be employed, e.g. using double sided adhesive tape. Stitching may be provided along critical edges or at specific points where additional reinforcement is needed.

The illustrated assembly 10 comprises just a top layer 15, an absorbent layer 14 and moisture barrier layer 12, but it will be understood that any number of additional layers may be provided depending upon requirements. Thus, further absorbent layers may be provided with different sizes and absorbent characteristics. Wicking layers and distribution layers may also be provided. Any layers between the top layer 15 and the barrier layer 12 may be termed intermediate layers.

The top layer 15 comprises a water-permeable material thus allowing the body fluids to migrate to the underlying absorbent layer 14. The top layer 15 may be constructed of any suitable fabric, including naturally derived fibres selected from the group consisting of cotton, wool, silk, cellulose, regenerated cellulose, rayon, viscose, modal, lyocell, Tencel, bamboo, hemp, flax, ramie, coir, or banana. Alternatively, the top layer may be constructed of synthetic fibres selected from the group consisting of polyamide, acrylic, polyester, or elastane, such as a mixture of polyester and elastane. Further, the top layer 15 may be constructed of a blend or a mixture of naturally derived and/or synthetic fibres. The materials used for construction of the top layer should be soft and non-irritating to the skin and be readily penetrated by any body fluids. The top layer is washable and may be of a knitted material. The top layer 15 may have an area weight of 80-200 gsm.

The absorbent layer 14 in the illustrated example is a washable weft knitted material having a first surface having a loop structure and a second surface opposite said first surface having rows of V-shaped stitches. The absorbent material comprises 70% by weight viscose, 18% by weight cotton and 12% by weight polyester. The first surface, being the loop surface, comprises viscose yarn and the second surface comprises a yarn of cotton and polyester. The loops have mainly a horizontal orientation on the material. The knitted absorbent material is a one-sided single knit stretch-Terry. The layer of absorbent material has a basis weight of 280 g/m². The absorbent material of viscose, cotton and polyester is without functional treatments such as wetting agents, softening agents, anti-odor treatments, optical brighteners, and biocidal agents. The yarns include spun yarns of short staple fibres. The yarn count is 45-65 S. One yarn is of viscose and the second yarn of cotton/polyester 60:40 in yarn.

The moisture barrier layer 12 may comprise a film or laminate e.g. of extruded polymeric material or a woven or knitted material of any suitable construction to prevent liquid from migrating from the assembly 10 to the fabric panel 2. The moisture barrier layer 12 in the illustrated variant, is a tightly woven activated carbon cloth that is sufficiently impermeable by nature. One suitable material is Flexzorb ^{®} from Chemviron SA. Laminates with polymer films may also be used. The activated carbon material may be highly desirable in reducing odours. The moisture barrier layer 12 may also comprise a coating of a moisture-impermeable material. The coating may be a polymer such as urethane wax or polyurethane provided for example on the surface facing away from the wearer of the moisture barrier layer 12.

The moisture barrier layer 12 is also preferably breathable, to allow vapour to escape from the absorbent undergarment 1 while preventing liquid from passing through the fabric layer. Air and vapour permeability may be achieved by the woven or knitted material or by an additional rate controlling layer attached thereto.

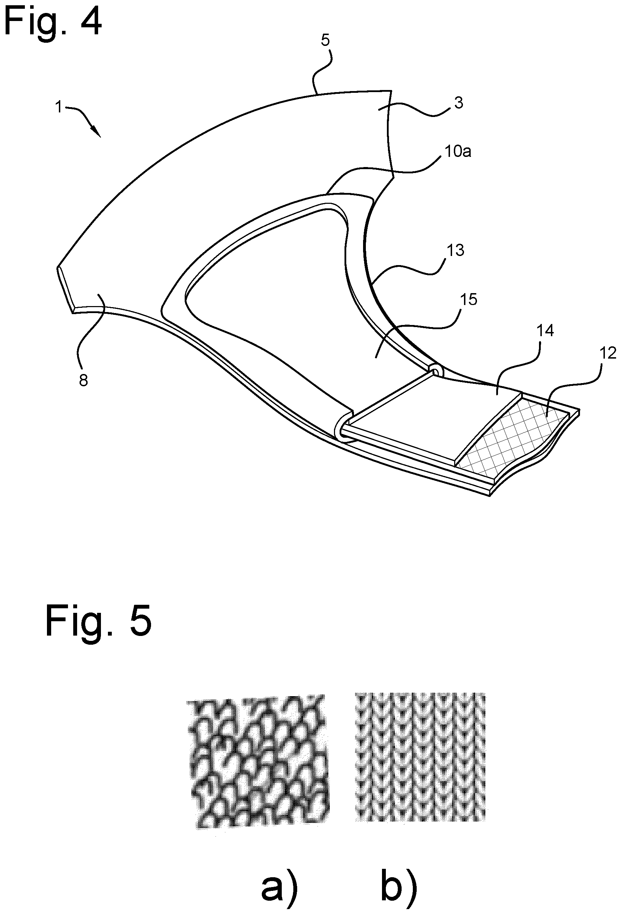

Fig. 4 shows part of the garment 1 in cutaway perspective view, viewed towards an inside surface 8 of the front side 3. The top layer 15 is partially removed to reveal the absorbent layer 14 and the moisture barrier layer 12 beneath. As can be seen in Fig. 5, which is a detail of Fig. 4, the absorbent layer 14 is provided with loops on its wearer facing side, as shown in Fig. 5a, and V-shaped stitches, as shown in Fig. 5b, on the garment facing side.

### Example

### FSC and CRC measurements

Absorption properties were evaluated by a method partly derived from the EDANA and INDA standard methods NWSP 240.0.R2 (commonly known as the Free Swell Capacity or FSC test) and NWSP 241.0.R2 (commonly known as the Centrifuge Retention Capacity or CRC test).

Representative samples are punched from the knitted fabrics under investigation. The samples should be disc shaped and have an area of about 10 cm². Before testing, the samples are conditioned for 24 hours in an environment set to 23°C and 50% relative humidity (subsequent testing takes place in this same environment). The sample is weighed to the third decimal. The sample is then placed in a tea bag like pouch, measuring about 60 x 60 mm, made from a heat sealable, porous web. The edges of the pouch are then closed using a heat sealer. The pouch is submerged into an excess amount of 0.9% (by mass) NaCl-solution. After a 5-minute absorption time the pouch is removed. The pouch is then hanged diagonally in a clamp from one of the double sealed corners, so that excess fluid can drip off. After a 2-minute drip time, a first weight is noted to the third decimal. The pouch is then placed in a centrifuge delivering a centrifugal acceleration of 250 g for a period of 3 minutes. After centrifugation, a second weight is noted to the third decimal. Suitably two pouches containing samples are tested at a time. However, the absorbency of the sample must be corrected for the weight and saline retention of the pouch web material, why two blank (empty) pouches can be tested and weighed along with the pouches containing the sample. In case of repeated testing, absorbency of the pouch web material suitably can be established beforehand. Free Swell Capacity (FSC) is determined after the 2-minute drip off time. The amount of saline in the sample is obtained by taking the first noted weight, and then subtracting the weight of the dry sample and the weight of a wet pouch web material. The amount of absorbed saline in the sample is then divided by the weight of the dry sample, to obtain an FSC absorbency on a g/g basis. Centrifuge Retention Capacity (CRC) is determined after centrifugation. The amount of saline in the sample is obtained by taking the second noted weight, and then subtracting the weight of the dry sample and the weight of a centrifugated pouch web material. The amount of absorbed saline in the sample is then divided by the weight of the dry sample, to obtain an CRC absorbency on a g/g basis.

Four knitted material samples with different fiber compositions as outlined in Table 1 were tested according to the above procedure. The knitted materials are weft knits having a first surface having a loop structure. The result of the test is shown in the same Table.

**Table 1**

| Sample | Fiber composition of knitted material | Weight (gsm) | FSC 5 min (g/g) | CRC (g/g) |
|---|---|---|---|---|
| A1 | 77% viscose, 14% cotton, 9% polyester | 395 | 5.0 | 1.5 |
| A2 | 70% viscose, 18 % cotton, 12 % polyester | 280 | 5.0 | 1.4 |
| B | 100 % polyester | 392 | 5.2 | 0.6 |
| C | 90 % cotton, 10 % polyester | 240 | 5.1 | 0.9 |

| | | | | |
|---|---|---|---|---|
| FSC- tests the absorbent capacity. CRC-tests the ability to keep the absorbed liquid. | | | | |

Table 1 shows that Sample A1 and A2 of knitted absorbent material according to the present disclosure has the highest combined inlet and rewet values, resulting in improved product performance related to liquid handling. It is believed that the loop structure of viscose improves the retention of the liquid and the cotton/polyester structure of the technical face of the material improves the absorption and spreading of the added liquid. The other tested materials in the test (B and C) show lower values for one of the two tested values leading to an overall poorer product performance related to liquid handling.

The disclosure may be varied within the scope of the appended claims. For example, the materials and dimensions used for the different layers forming the absorbent assembly may be varied, as indicated herein.

## Claims

1. A washable integrated absorbent assembly for an absorbent article, the assembly extending in a longitudinal direction and having a transverse direction, and comprising an absorbent layer and a liquid barrier, the absorbent layer being of a knitted material having a first and a second surface, the first surface having a loop structure, wherein the knitted absorbent material comprises a major amount of viscose and further comprises cotton and polyester.

2. Absorbent assembly according to claim 1, wherein the knitted absorbent material comprises at least 60% by weight viscose.

3. Absorbent assembly according to claim 1 or 2, wherein the knitted absorbent material comprises 60-85% by weight viscose, 10-25 % by weight cotton and 5-20 % by weight polyester.

4. Absorbent assembly according to anyone of the claims 1-3, wherein the knitted absorbent material comprises 65-80% viscose, 10-20% cotton, 5-15% polyester.

5. Absorbent assembly according to anyone of the claims 1-4, wherein the knitted absorbent material comprises a first yarn comprising viscose and a second yarn comprising cotton and polyester.

6. Absorbent assembly according to anyone of the claims 1-5, wherein the loop structure is of a yarn comprising viscose.

7. Absorbent assembly according to anyone of the claims 1-6, wherein the second surface has rows of V-shaped stitches.

8. Absorbent assembly according to anyone of the claims 1-7, wherein the knitted absorbent material is a weft knit or a circular knit.

9. Absorbent assembly according to anyone of the claims 1-8, wherein the knitted absorbent material has a basis weight of 180-600 g/m².

10. Absorbent assembly according to anyone of the claims 1-9, wherein the loop structure has loops having a mainly horizontal orientation on the material.

11. Absorbent assembly according to anyone of the claims 1-10, wherein the knitted absorbent material is a one-sided or double-sided stretch-Terry.

12. Absorbent assembly according to anyone of the claims 1-11, wherein the first surface of the knitted absorbent material comprises viscose and the second surface comprises cotton and polyester.

13. Absorbent assembly according to anyone of the claims 1-12, wherein the assembly further comprises a wearer facing top layer.

14. Washable absorbent underwear comprising an absorbent assembly according to any of the preceding claims permanently attached to a fabric panel of the underwear.
